# EUROPÄISCHE PATENTANMELDUNG

(11) **EP 3 753 490 A1**
(43) Veröffentlichungstag der Anmeldung: **23.12.2020**
(21) Anmeldenummer: 19181126.4
(22) Anmeldetag: 19.06.2019
(51) Int. Cl.: A61B 5/145, A61B 5/1455, A61B 5/1477

(54) **MEMBRAN ZUR TRANSKUTANEN BLUTGASMESSUNG**

(71) Anmelder: Axenoll Life Sciences AG, 8126 Zumikon (CH)
(72) Erfinder: VASIC, Srdan, 07745 Jena (DE); SCHMITT, Rafael, 07745 Jena (DE)
(74) Vertreter: Wenzel Nemetzade Warthmüller Patentanwälte Part mbB

(57) **Zusammenfassung**

Membran zur transkutanen Blutgasmessung, mit wenigstens einem Gasdurchleitungsabschnitt, der durch eine poröse Struktur gebildet ist.

## Beschreibung

Die vorliegende Erfindung betrifft eine Membran zur transkutanen Blutgasmessung. Ebenso betrifft die vorliegende Erfindung einen Drucknutzen mit einer Mehrzahl von Membranen, einen Sensor mit einer solchen Membran sowie ein Verfahren zur Herstellung einer Membran.

Es ist bekannt, mittels einer Blutgasanalyse die Gasverteilung beziehungsweise den Partialdruck von Sauerstoff und/oder Kohlenstoffdioxid zu messen. Mittels einer Blutgasanalyse kann insbesondere die Blutsauerstoffsättigung gemessen werden. Ebenso können mittels Blutgasanalyse pH-Wert, Standardbicarbonat und/oder Basenüberschuss gemessen werden. Dabei ist es möglich, Blutgasmessungen transkutan vorzunehmen. Insbesondere bei der Überwachung von Neugeborenen kommt eine transkutane Blutgasmessung zum Einsatz, bevorzugt zur Messung der Partialdrücke von Sauerstoff und/oder Kohlenstoffdioxid.

Für eine transkutane Blutgasmessung werden Sensoren mit einer gasdurchlässigen Membran eingesetzt. Am Außenumfang einer solchen Membran können Befestigungsabschnitte zur Befestigung der Membran innerhalb des Sensorkopfes vorgesehen sein.

Solche Membranen können mittels eines Lasers ausgeschnitten werden, wobei die Gasdurchlässigkeit durch eine verhältnismäßig hohe Anzahl an gleichartigen beziehungsweise regelmäßig zueinander angeordneten Bohrungen in der Membran gewährleistet werden kann. Durch das nachträgliche Einbringen von Bohrungen in eine solche Membran entsteht ein verhältnismäßig hoher Aufwand in der Herstellung. Zudem gewährleisten Bohrungen in der Membran eine nur begrenzte Gasdurchlässigkeit. Es sind daher verhältnismäßig viele Bohrungen erforderlich, um ein Mindestmaß an Gasdurchlässigkeit zu erreichen, wodurch wiederum der Herstellaufwand vergrößert wird.

Vor dem oben dargelegten Hintergrund bestand die Aufgabe der vorliegenden Erfindung darin, eine Membran zur transkutanen Blutgasmessung anzugeben, die mit geringem Aufwand hergestellt werden kann und gleichzeitig ein hohes Maß an Gasdurchlässigkeit aufweist. Ebenso bestand die Aufgabe darin, einen Sensor zur transkutanen Blutgasmessung sowie ein Verfahren zur Herstellung einer Membran zur transkutanen Blutgasmessung anzugeben.

In Bezug auf die Membran ist diese Aufgabe mit dem Gegenstand des Anspruchs 1 gelöst worden. Ein erfindungsgemäßer Sensor ist Gegenstand von Anspruch 19 und ein Verfahren zur Herstellung einer Membran ist in Anspruch 20 angegeben.

Eine erfindungsgemäße Membran zur transkutanen Blutgasmessung weist wenigstens einen Gasdurchleitungsabschnitt auf, der durch eine poröse Struktur gebildet ist.

Unter der Bezeichnung "transkutan" soll hier "durch die Haut hindurch", insbesondere "durch die Haut hindurch ohne Verletzung der Haut" beziehungsweise "nicht-invasiv" verstanden werden. Eine transkutane Blutgasmessung im Sinne der vorliegenden Lehre erfolgt also "durch die Haut hindurch", insbesondere "durch die Haut hindurch ohne Verletzung der Haut" beziehungsweise "nicht-invasiv".

Mit dem Begriff "porös" sollen allgemein Strukturen bezeichnet werden, die eine Vielzahl von Hohlräumen aufweisen. Solche Hohlräume können beispielsweise als Poren und/oder Kavitäten ausgebildet sein. Im Sinne der vorliegenden Erfindung kann es sich bei solchen Hohlräumen, Poren beziehungsweise Kavitäten um sehr kleine Öffnungen, Höhlungen und/oder Vertiefungen handeln.

Die Porosität kann dabei das Verhältnis des Hohlraumvolumens relativ zu dem gesamten Festkörpervolumen beziehungsweise relativ zu dem Volumen des jeweiligen Festkörperabschnitts bezeichnen. Die Porosität ist somit ein Maß für das Hohlraumvolumen innerhalb eines Festkörpers oder einer Struktur.

Im Sinne der vorliegenden Erfindung sollen durch die Angabe "porös" im speziellen Strukturen bezeichnet werden, in denen zumindest einige Hohlräume beziehungsweise Poren entlang einer Dickenrichtung der jeweiligen Struktur verteilt angeordnet sind und/oder geneigt zu einer Dickenrichtung der jeweiligen Struktur verlaufen und/oder entlang einer Dickenrichtung der jeweiligen Struktur eine sich verändernde Querschnitts- und/oder Längsschnittform aufweisen und/oder allgemein unterschiedliche Formgebungen aufweisen. Im Übrigen können Hohlräume oder Poren einer porösen Struktur im Sinne der vorliegenden Erfindung beliebig ausgebildet, angeordnet und/oder verteilt sein.

Im Folgenden wird zur Erläuterung der Erfindung sowie vorteilhafter Ausgestaltungen die allgemeine Bezeichnung "Hohlraum" verwendet, wobei sich die jeweiligen Erläuterungen auch auf Poren, Kavitäten, Öffnungen, Höhlungen und/oder Vertiefungen beziehen.

Unter einer porösen Struktur kann im Sinne der vorliegenden Erfindung insbesondere eine poröse Festkörperstruktur verstanden werden. Eine solche Festkörperstruktur kann steif oder flexibel verformbar ausgebildet sein.

Durch eine poröse Struktur kann ein verhältnismäßig hohes Maß an Gasdurchlässigkeit gewährleistet werden. Zudem kann durch eine poröse Struktur entlang des gesamten Gasdurchleitungsabschnitts eine verhältnismäßig homogene Gasdurchlässigkeit gewährleistet werden. Nachträglich eingebrachte Bohrungen in einer Membran können demgegenüber eine nur punktuelle beziehungsweise inhomogenere Gasdurchlässigkeit gewährleisten.

Schließlich besteht durch das Vorsehen einer porösen Struktur die Möglichkeit, die Gasdurchlässigkeit mittels geeigneter Herstellparameter gezielt einzustellen. Insbesondere kann die Gasdurchlässigkeit im Hinblick auf die jeweiligen Einsatzbedingungen beziehungsweise Anforderungen in geeigneter Weise ausgewählt und dann in hoher Genauigkeit eingestellt werden. Die Einstellung der Gasdurchlässigkeit kann insbesondere über die Porosität erfolgen.

Schließlich können durch den Gasdurchleitungsabschnitt, welcher durch eine poröse Struktur gebildet ist, Nachbearbeitungen vermieden werden. Der Herstellaufwand wird auf diese Weise verringert. Insbesondere kann der Aufwand für das nachträgliche Einbringen von Bohrungen entfallen.

Eine poröse Struktur kann im Quer- und/oder Längsschnitt des Gasdurchleitungsabschnitts unterschiedliche geometrische Formen annehmen. Insbesondere kann die Porosität durch unterschiedlich ausgebildete Formgebungen im Inneren des Gasdurchleitungsabschnitts gebildet beziehungsweise erzeugt sein.

Im Sinne der vorliegenden Erfindung soll eine poröse Struktur nicht begrenzt sein auf Strukturen mit kreisrunden beziehungsweise kugelförmigen Hohlräumen. Vielmehr können die Hohlräume einer solchen porösen Struktur beliebig geformt sein.

Eine poröse Struktur kann beispielsweise eckige, zylinderförmige, kegelförmige, pyramidenförmige, ellipsoide, quaderförmige, würfelförmige und/oder unregelmäßige Hohlräume aufweisen. Ebenso ist es möglich, dass die poröse Struktur Hohlräume unterschiedlicher Formgebungen aufweist. Beispielsweise besteht die Möglichkeit, dass die poröse Struktur sowohl kugelförmige als auch ellipsoide Hohlräume aufweist.

Gemäß einer weiteren vorteilhaften Ausgestaltung der vorliegenden Erfindung können Hohlräume unterschiedlicher Größendimensionen vorgesehen sein. Beispielsweise können Hohlräume vorhanden sein, deren Größenverhältnisse sich um den Faktor 2 unterscheiden. Weiterhin können sich die Größen der Hohlräume um einen Faktor von bis zu 3, bis zu 4, bis zu 5, bis zu 6, bis zu 7, bis zu 8, bis zu 9 oder bis zu 10 unterscheiden. Solche Größenverhältnisse können beispielsweise mikroskopisch beziehungsweise mittels eines Rasterelektronenmikroskops erfasst werden.

Gemäß einer vorteilhaften Ausgestaltung der vorliegenden Erfindung kann der Gasdurchleitungsabschnitt durch eine poröse Metallstruktur gebildet sein. Eine solche poröse Metallstruktur kann mit nur geringem Aufwand hergestellt werden und gewährleistet gleichzeitig ein hohes Maß an Betriebssicherheit. Insbesondere weisen poröse Metallstrukturen eine verhältnismäßig geringe Neigung zur Riss- oder Bruchbildung auf.

Gemäß einer weiter vorteilhaften Ausgestaltung der vorliegenden Erfindung kann der Gasdurchleitungsabschnitt durch eine poröse Keramikstruktur gebildet sein. Durch ein keramisches Material kann eine verhältnismäßig lange Einsatzdauer und gleichzeitig auch eine gute Hautverträglichkeit der Membran sichergestellt werden.

Gemäß einer vorteilhaften Ausgestaltung der vorliegenden Erfindung kann der Gasdurchleitungsabschnitt durch eine Struktur mit offener Porosität und/oder durch eine offenzellig poröse Struktur gebildet sein. Eine derartige offene Porosität beziehungsweise offenzellig poröse Struktur ermöglicht ein besonders hohes Maß an Gasdurchlässigkeit. Es kann sich insbesondere um eine offenzellige Struktur beziehungsweise Metallstruktur mit definierter und/oder homogener oder inhomogener Porosität handeln

In bevorzugter Weise können einzelne Hohlräume einer porösen Struktur miteinander in Fluidverbindung stehen, so dass entlang der einzelnen Hohlräume der Struktur ein Hindurchtreten eines gasförmigen Mediums in jeweils gewünschtem Umfang erfolgen kann.

Ebenso ist es möglich, dass der Gasdurchleitungsabschnitt durch eine Struktur mit geschlossener beziehungsweise teilweise geschlossener Porosität und/oder durch eine geschlossenzellig poröse beziehungsweise teilweise geschlossenzellig poröse Struktur gebildet ist. Durch eine geschlossene beziehungsweise teilweise geschlossene Porosität und/oder geschlossenzellig poröse Struktur kann die Gasdurchlässigkeit in geeigneter Weise begrenzt werden.

Ferner ist es gemäß einer bevorzugten Ausgestaltung möglich, dass der Gasdurchleitungsabschnitt durch eine Struktur mit einer teilweise offenen Porosität und/oder mit einer teilweise geschlossenen Porosität gebildet ist.

Gemäß einer weiter bevorzugten Ausführungsform können Hohlräume der porösen Struktur Negativformen von nachträglich entfernten Formgebungselementen abbilden. Es besteht demnach die Möglichkeit durch Auswahl geeigneter Formgebungselemente in der jeweils gewünschten Form, Größe, Anzahl, Verteilung und/oder Dichte Einfluss auf die räumlich-körperliche Ausprägung der porösen Struktur zu nehmen. Die Gasdurchlässigkeit des Gasdurchleitungsabschnitts kann auf diese Weise präzise eingestellt beziehungsweise im Hinblick auf die jeweiligen Anforderungen ausgewählt werden.

Gemäß einer weiteren vorteilhaften Ausgestaltung kann die poröse Struktur hergestellt sein durch Erzeugung eines Materialabschnitts aus einem metallischen oder keramischen Matrixwerkstoff mit darin angeordneten Formgebungselementen aus Kunststoff und durch Erhitzen des Materialabschnitts zur nachträglichen Entfernung der Formgebungselemente. Durch einen metallischen oder keramischen Matrixwerkstoff kann einerseits eine ausreichende Stabilität gewährleistet werden. Das Anordnen von Formgebungselementen aus Kunststoff ermöglicht andererseits ein verhältnismäßig einfaches nachträgliches Entfernen dieser Formgebungselemente durch Erhitzen. Auf diese Weise kann mit nur geringem Aufwand eine poröse Metallstruktur oder eine poröse Keramikstruktur mit den jeweils gewünschten geometrischen Eigenschaften erzeugt werden. Zudem können Formgebungselemente aus Kunststoff mit geringem Kostenaufwand und mit verhältnismäßig hoher geometrischer Präzision bereitgestellt und somit zur Herstellung einer erfindungsgemäßen Membran verwendet werden.

In vorteilhafter Weise kann es sich bei dem Matrixwerkstoff um eine Fe-Basislegierung, einen Stahlwerkstoff beziehungsweise eine Stahllegierung oder um einen Titanwerkstoff beziehungsweise eine Titanlegierung handeln. Ebenso kann es sich bei dem Matrixwerkstoff um eine Keramiklegierung handeln.

In weiter vorteilhafter Weise können die voranstehend erwähnten Formgebungselemente aus einem Polymerwerkstoff bestehen. Auf diese Weise kann deren Entfernung auf besonders einfache Weise durch Erhitzen erfolgen.

Gemäß einer weiter bevorzugten Ausgestaltung kann die poröse Struktur gesintert sein und/oder die Porosität durch Sintern erzeugt sein. Das Sintern einer porösen Struktur beziehungsweise die Erzeugung einer Porosität durch Sintern kann mit nur geringem Aufwand und verhältnismäßig hoher Genauigkeit bewerkstelligt werden.

Gemäß einer weiter bevorzugten Ausgestaltung kann die poröse Struktur durch Sintern eines metallischen oder keramischen Matrixwerkstoffs mit darin angeordneten Formgebungselementen erzeugt sein. Ferner können die Formgebungselemente durch Ausschmelzen, Ausbrennen und/oder Ausgasen entfernt sein. In besonders vorteilhafter Weise können die Formgebungselemente durch gleichzeitiges Sintern eines metallischen oder keramischen Matrixwerkstoffs entfernt sein.

Es kann also der metallische oder keramische Matrixwerkstoff durch Sintern in seiner Form eine Verfestigung erfahren und gleichzeitig das Kunststoffmaterial der Formgebungselemente aufgrund des Sinterprozesses erhitzt werden. Durch ein derartiges Erhitzen können die Formgebungselemente ausschmelzen, ausbrennen und/oder ausgasen. Dies ermöglicht eine besonders effiziente Herstellung, da die Verfestigung des metallischen oder keramischen Matrixwerkstoffs gleichzeitig mit der Entfernung der Formgebungselemente umgesetzt werden kann. Eine im Nachgang zum Sintern des metallischen oder keramischen Matrixwerkstoffs vorzunehmende Bearbeitung kann somit entfallen.

Gemäß einer weiter bevorzugten Ausgestaltung kann die poröse Struktur durch Sintern eines metallischen oder keramischen Werkstoffs frei von gesonderten Formgebungselementen erzeugt sein. Die poröse Struktur kann demnach auch ohne den Einsatz von gesonderten Formgebungselementen innerhalb eines Matrixwerkstoffs hergestellt werden. Dabei besteht beispielsweise die Möglichkeit, über die Sintertemperatur und/oder Sinterdauer Einfluss auf die Porosität der erzeugten Struktur zu nehmen. Ebenfalls kann über die Auswahl geeigneter Sinterwerkstoffe beziehungsweise Werkstoffzusammensetzung die Porosität eingestellt werden.

Gemäß einer weiter bevorzugten Ausgestaltung kann der Gasdurchleitungsabschnitt durch additive Fertigung erzeugt sein, insbesondere durch 3D-Siebruck. Durch den Einsatz eines solchen Fertigungsverfahrens kann ein hohes Maß an Flexibilität gewährleistet werden. Insbesondere können mittels eines solchen Fertigungsverfahrens komplexe geometrische Strukturen mit nur geringem Aufwand erzeugt werden. Bei dem Einsatz der additiven Fertigung, insbesondere dem 3D-Siebdruck, bestehen nur geringe Restriktionen an die geometrische Gestaltung. Ferner ermöglicht der Einsatz eines additiven Fertigungsverfahrens, insbesondere die 3D-Siebdrucktechnologie, ein hohes Maß an Fertigungsproduktivität.

Gemäß einer weiter bevorzugten Ausgestaltung kann der Gasdurchleitungsabschnitt aus einer Druckpaste mit Formgebungselementen aus Kunststoff gedruckt und nachträglich gesintert sein. Durch den Einsatz einer derartigen Druckpaste können die Eigenschaften des Gasdurchleitungsabschnitts in geeigneter Weise eingestellt werden. So kann je nach Anwendungsfall eine andere Druckpaste für die Erzeugung des Gasdurchleitungsabschnitts gewählt werden, beispielsweise eine Druckpaste mit einer geeignet ausgewählten Dichte an Formgebungselementen, geometrischen Gegebenheiten der Formgebungselementen beziehungsweise auch mit unterschiedlichen Matrixwerkstoffen. Die Flexibilität in der Herstellung des Gasdurchleitungsabschnitts wird auf diese Weise weiter verbessert.

Ebenso ist es möglich, eine Druckpaste frei von Formgebungselementen für die Herstellung des Gasdurchleitungsabschnitts zu verwenden. In diesem Fall kann durch eine geeignete Pastenzusammensetzung und/oder geeigneter Sinterparameter, wie zum Beispiel Sintertemperatur und/oder Sinterdauer, die Porosität eingestellt werden.

In weiter bevorzugter Ausgestaltung kann der Gasdurchleitungsabschnitt durch eine Mehrzahl an Schichten erzeugt sein. Insbesondere kann der Gasdurchleitungsabschnitt im 3D-Siebdruckverfahren durch schichtweisen Materialauftrag einer Druckpaste erzeugt sein. Zwischen den jeweiligen Druckvorgängen zur Erzeugung einer Druckschicht kann eine Trocknung vorgenommen werden. Nach Abschluss des schichtweisen Aufbaus kann dann eine Sinterung erfolgen, durch die der Matrixwerkstoff verfestigt und die Formgebungselemente aus dem Matrixwerkstoff entfernt werden.

In weiter bevorzugter Weise kann zumindest eine Außenabmessung, insbesondere der Außendurchmesser, die Dicke und/oder die Länge, eines Formgebungselements in der Druckpaste relativ zur Dicke einer Drucklage kleiner, gleich oder größer bemessen sein.

Gemäß einer weiter bevorzugten Ausgestaltung ist der Gasdurchleitungsabschnitt permeabel, zumindest für Sauerstoff und/oder Kohlenstoffdioxid permeabel. In besonders bevorzugter Weise kann der Gasdurchleitungsabschnitt für aus der menschlichen Haut austretenden Sauerstoff und/oder Kohlenstoffdioxid permeabel sein. Auf diese Weise kann die Membran im Anwendungsfall auf der Haut eines Patienten beziehungsweise einer zu überwachenden Person angebracht werden. Nach dem Anbringen auf der Haut können in geeigneter Weise Messungen des Sauerstoffpartialdrucks beziehungsweise des Kohlenstoffdioxidpartialdrucks vorgenommen werden.

In weiter bevorzugter Weise kann der Gasdurchleitungsabschnitt eine eingestellte Porosität aufweisen, insbesondere eine Porosität von 0,1 bis 0,9, bevorzugt von 0,2 bis 0,8, weiter bevorzugt von 0,3 bis 0,7, noch weiter bevorzugt von 0,4 bis 0,6. Eine solche Porosität kann sich in vorteilhafter Weise für die Durchleitung von gasförmigen Medien erweisen.

Ferner kann die Porosität des Gasdurchleitungsabschnitts durch die Konzentration von Formgebungselementen eingestellt sein, insbesondere durch die Konzentration von Formgebungselementen in der jeweils eingesetzten Druckpaste. Beispielsweise kann eine Druckpaste mit einer hohen Konzentration von Formgebungselementen dazu führen, dass nach dem Entfernen der Formgebungselemente ein verhältnismäßig großes Hohlraumvolumen innerhalb des Gasdurchleitungsabschnitts entsteht. Demgegenüber kann eine geringere Konzentration von Formgebungselementen zu einem geringeren Hohlraumvolumen des Gasdurchleitungsabschnitts führen. Unterschiedliche Hohlraumvolumina haben wiederum Einfluss auf die Porosität des Gasdurchleitungsabschnitts, wodurch die Gasdurchleitungseigenschaften in geeigneter Weise eingestellt beziehungsweise ausgewählt werden können.

In besonders bevorzugter Weise kann der Gasdurchleitungsabschnitt eine eingestellte Permeabilität aufweisen. Eine jeweils eingestellte Permeabilität erlaubt in besonders geeigneter Weise das Hindurchtreten von Sauerstoff und/oder Kohlenstoffdioxidgasen. Eine derartige Membran mit einem solchen Gasdurchleitungsabschnitt kann in besonders vorteilhafter Weise für die transkutane Blutgasmessung eingesetzt werden.

Gemäß einem weiteren Ausführungsbeispiel der vorliegenden Erfindung kann die Permeabilität des Gasdurchleitungsabschnitts durch die Konzentration von Formgebungselementen eingestellt sein. Beispielsweise kann eine Druckpaste mit einer hohen Konzentration von Formgebungselementen dazu führen, dass nach dem Entfernen der Formgebungselemente ein verhältnismäßig großes Hohlraumvolumen innerhalb des Gasdurchleitungsabschnitts entsteht. Demgegenüber kann eine geringere Konzentration von Formgebungselementen zu einem geringeren Hohlraumvolumen des Gasdurchleitungsabschnitts führen. Unterschiedliche Hohlraumvolumina können wiederum Einfluss auf die Permeabilität des Gasdurchleitungsabschnitts haben, wodurch die Gasdurchleitungseigenschaften in geeigneter Weise eingestellt beziehungsweise ausgewählt werden können.

Gemäß einer weiteren Ausführungsform kann der Gasdurchleitungsabschnitt eine Dicke von 0,01 mm bis 1 mm, bevorzugt von 0,02 mm bis 0,08 mm, weiter bevorzugt von 0,03 mm bis 0,07 mm, noch weiter bevorzugt von 0,04 mm bis 0,06 mm, insbesondere etwa 0,05 mm aufweisen. Ein Gasdurchleitungsabschnitt mit einer solchen Dicke kann einerseits eine ausreichende mechanische Stabilität gewährleisten und gleichzeitig die gewünschten Permeabilitäts- beziehungsweise Durchlässigkeitseigenschaften sicherstellen.

In weiter bevorzugter Ausgestaltung kann der Gasdurchleitungsabschnitt einen Durchmesser und/oder eine Diagonallänge von 0,5 mm bis 1,5 mm, bevorzugt von 0,7 mm bis 1,3 mm, weiter bevorzugt von 0,9 mm bis 1,1 mm, insbesondere etwa 1 mm aufweisen. Derartige Abmessungen ermöglichen in vorteilhafter Weise den Einsatz innerhalb eines kompakten Sensorkopfes. Der Einsatz für die transkutane Blutgasmessung von Neugeborenen wird auf diese Weise besonders begünstigt.

Gemäß einer weiteren vorteilhaften Ausgestaltung kann zumindest ein Befestigungsabschnitt zur Befestigung des Gasdurchleitungsabschnitts an einem Sensor zur transkutanen Blutgasmessung vorgesehen sein. Ebenso ist es möglich, dass eine Mehrzahl von Befestigungsabschnitten vorgesehen ist. Durch einen solchen Befestigungsabschnitt kann insbesondere die stabile Fixierung des Gasdurchleitungsabschnitts innerhalb eines Sensorkopfes gewährleistet werden, ohne dass die Gasdurchleitungseigenschaften des Gasdurchleitungsabschnitts beeinträchtigt werden.

Dabei kann ein Befestigungsabschnitt in vorteilhafter Weise einstückig mit dem Gasdurchleitungsabschnitt ausgebildet sein. Ein solcher Befestigungsabschnitt oder eine Mehrzahl von Befestigungsabschnitten kann somit zusammen mit dem Gasdurchleitungsabschnitt hergestellt werden, also im selben Fertigungsprozess. Beispielsweise können im 3D-Siebdruck der Gasdurchleitungsabschnitt sowie der zumindest eine Befestigungsabschnitt gleichzeitig schichtweise erzeugt werden. Hierdurch kann ein hohes Maß an Fertigungseffizienz gewährleistet werden.

Es ist weiterhin möglich, dass der Befestigungsabschnitt an den Gasdurchleitungsabschnitt angrenzt. Ferner kann der Befestigungsabschnitt auch von dem Gasdurchleitungsabschnitt beabstandet angeordnet sein beziehungsweise kann zwischen dem Befestigungsabschnitt und dem Gasdurchleitungsabschnitt noch ein weiterer Abschnitt oder eine Unterbrechung vorgesehen sein. Die Gestaltungsflexibilität wird auf diese Weise weiter verbessert.

In weiter bevorzugter Weise kann eine Mehrzahl an Befestigungsabschnitten um den Gasdurchleitungsabschnitt herum angeordnet sein, insbesondere gleichmäßig um den Gasdurchleitungsabschnitt herum angeordnet sein. Es kann auf diese Weise mittels der Befestigungsabschnitte eine stabile und gleichmäßige Befestigung des Gasdurchleitungsabschnitts gewährleistet werden, wodurch sich die Funktionssicherheit verbessern lässt.

Gemäß einer weiter bevorzugten Ausgestaltung kann der Befestigungsabschnitt relativ zum Gasdurchleitungsabschnitt knickbar und/oder biegbar sein, insbesondere zerstörungsfrei relativ zum Gasdurchleitungsabschnitt knickbar und/oder biegbar sein. Durch eine solche knickbare beziehungsweise biegbare Ausgestaltung kann mit nur geringem manuellem Handhabungsaufwand oder auch automatisierten Aufwand eine kraft- und/oder formschlüssige Verbindung zwischen dem Gasdurchleitungsabschnitt und einem Sensorkopf beziehungsweise einem die Membran tragenden Sensor gewährleistet werden. Ebenso kann aufgrund der knickbaren beziehungsweise biegbaren Ausgestaltung ein einfaches Lösen der Membran ermöglicht werden, insbesondere ohne dass hierfür Komponenten zerstört werden müssten.

Gemäß einer weiter bevorzugten Ausgestaltung kann der Befestigungsabschnitt gegenüber dem Gasdurchleitungsabschnitt vorstehen. Insbesondere kann der wenigstens eine Befestigungsabschnitt gegenüber dem Gasdurchleitungsabschnitt in einer ungeknickten und/oder unverbogenen Ausgangsposition gegenüber einer Außenkontur des Gasdurchleitungsabschnitts vorstehen und/oder in einer geknickten oder gebogenen Stellung in Dickenrichtung gegenüber dem Gasdurchleitungsabschnitt vorstehen. Es kann auf diese Weise sichergestellt werden, dass die Befestigung des Gasdurchleitungsabschnitts mittels des Befestigungsabschnitts eine Verformung durch Knicken und/oder Biegen entlang eines Sollbereichs zur Folge hat. Insbesondere können hierdurch unerwünschte Verformungen des Gasdurchleitungsabschnitts selbst vermieden werden. Eine Verformung kann hingegen an dem Befestigungsabschnitt erfolgen. Ebenso kann die Verformung im Grenzbereich zwischen dem Gasdurchleitungsabschnitt und dem jeweiligen Befestigungsabschnitt erfolgen.

Gemäß einer weiter bevorzugten Ausgestaltung kann der Befestigungsabschnitt L-förmig oder T-förmig ausgebildet sein und/oder zur formschlüssigen Befestigung und/oder zum Hintergreifen einer an einem Sensor vorgesehen Eingriffsstruktur ausgebildet sein. Durch eine derartige geometrische Ausgestaltung des Befestigungsabschnitts kann die Befestigungssicherheit und damit auch die Betriebssicherheit verbessert werden.

In weiter bevorzugter Weise kann der größtmögliche Abstand zwischen zwei freien Enden gegenüberliegender Befestigungsabschnitte zwischen 1 mm und 2 mm betragen, insbesondere zwischen 1,2 mm und 1,8 mm, bevorzugt etwa 1,5 mm. Eine derartige Dimensionierung stellt einerseits einen kompakten Aufbau der Membran sicher. Gleichzeitig wird jedoch eine ausreichende Erstreckung des Gasdurchleitungsabschnitts zwischen den gegenüberliegenden Befestigungsabschnitten gewährleistet beziehungsweise können die Befestigungsabschnitte selbst noch entlang eines ausreichend großen Streckenabschnitts ausgehend von dem Gasdurchleitungsabschnitt verlaufen, so dass diese in geeigneter Weise für die jeweilige Befestigungsaufgabe verwendet werden können.

Gemäß einer weiteren vorteilhaften Ausgestaltung kann der Gasdurchleitungsabschnitt unterbrechungsfrei in den Befestigungsabschnitt übergehen. Hierdurch kann die Herstellung der Membran vereinfacht werden. Dies gilt insbesondere für die Herstellung mittels 3D-Siebdruck. In diesem Fall können nämlich der Gasdurchleitungsabschnitt und der Befestigungsabschnitt gleichzeitig durch schichtweisen Aufbau erzeugt werden.

In besonders bevorzugter Weise können der Gasdurchleitungsabschnitt und der Befestigungsabschnitt eine identische Lagenstruktur aufweisen und/oder eine identische Dicke aufweisen. Besonders bevorzugt können der Gasdurchleitungsabschnitt und der Befestigungsabschnitt gemeinsam durch 3D-Siebdruck erzeugt sein. Dies kann in besonders effizienter Weise erfolgen, da die Lagen im 3D-Siebdruckverfahren durchgehend erzeugt werden können und somit der schichtweise Aufbau unabhängig von den unterschiedlichen Abschnitten der Membran vorgenommen werden kann.

Ein weiterer Aspekt der vorliegenden Erfindung betrifft einen Drucknutzen, insbesondere 3D-Siebdrucknutzen. Ein erfindungsgemäßer Drucknutzen weist einen Rahmenabschnitt und eine Mehrzahl von voranstehend beschriebenen Membranen auf. Dabei kann jeweils eine Membran durch zumindest einen Verbindungssteg mit dem Rahmenabschnitt verbunden sein.

Unter einem Drucknutzen kann allgemein die Fläche verstanden werden, die mittels eines 3D-Siebdruckprozesses, also durch einen Druckvorgang, bedruckt wird. Vorliegend soll unter Drucknutzen jedoch ein Erzeugnis verstanden werden, welches auf einer solchen Druckfläche durch schichtweisen Aufbau erzeugt wird. Der Drucknutzen bezeichnet somit im Sinne der vorliegenden Erfindung ein Erzeugnis, das durch eine Mehrzahl von Druckvorgängen auf einer zu bedruckenden Druckfläche entsteht. Es handelt sich bei der Druckfläche um eine Fläche, die in einem Druckvorgang bedruckt werden kann. Im Falle mehrerer nebeneinander angeordneter Druckflächen, die in unterschiedlichen Druckvorgängen bedruckt werden, entstehen mehrere Drucknutzen.

Ein erfindungsgemäßer Drucknutzen weist eine Vielzahl einzelner Membrane auf und kann somit mit besonders hoher Effizienz gefertigt werden. Insbesondere kann der gesamte Drucknutzen mit den jeweiligen Membranen schichtweise erzeugt werden, so dass durch einen solchen schichtweisen Aufbau im 3D-Siebdruckverfahren gleichzeitig eine Vielzahl einzelner Membrane erzeugt werden kann. Dabei gewährleistet der Rahmenabschnitt des Drucknutzens, dass die einzelnen Membrane sicher in ihrer Position untereinander beziehungsweise zum Rahmenabschnitt gehalten werden beziehungsweise in Position bleiben.

Ein erfindungsgemäßer Drucknutzen kann eine Größe aufweisen, die von einer 3D-Siebdruckmaschine in einem Druckvorgang bedruckt werden kann, wie voranstehend bereits erwähnt. Es kann sich beispielsweise um einen Drucknutzen handeln, der eine Länge von 60 bis 70 mm, insbesondere etwa 64 mm, und/oder eine Breite von 40 bis 50 mm, insbesondere etwa 44 mm aufweist. Ein erfindungsgemäßer Drucknutzen kann beispielsweise 10 bis 20 Reihen von Membranen, insbesondere 16 Reihen von Membranen und/oder 5 bis 15 Zeilen von Membranen, insbesondere 9 Zeilen von Membranen aufweist.

In weiter bevorzugter Weise kann ein Drucknutzen 50 bis 200 einzelner Membrane aufweisen, insbesondere 120 bis 180 einzelner Membrane, insbesondere 144 einzelner Membrane.

Gemäß einer weiter bevorzugten Ausgestaltung kann zumindest eine Membran durch wenigstens zwei Verbindungsstege mit dem Rahmenabschnitt verbunden sein. Derartige Verbindungsstege können bevorzugt an gegenüberliegenden Seiten beziehungsweise Enden der Membran vorgesehen sein. Es kann sich insbesondere um Enden in einer Flächenerstreckung handeln.

Derartige Verbindungsstege können weiter bevorzugt in Umfangsrichtung der Membran zwischen zwei benachbarten Verbindungsabschnitten angeordnet sein. Ein erfindungsgemäßer Verbindungssteg kann beispielsweise eine in Draufsicht gesehene Breite von 0,05 mm bis 0,15 mm, insbesondere 0,1 mm aufweisen.

In weiter bevorzugter Weise kann ein Verbindungssteg geradlinig, insbesondere frei von Krümmungen und/oder winkeligen Formgebungen, zwischen dem Rahmenabschnitt und der jeweiligen Membran verlaufen. Ebenso ist es möglich, dass der Verbindungssteg zwischen dem Rahmenabschnitt und der jeweiligen Membran gekrümmt, gezackt und/oder gewinkelt verläuft. Durch eine solche Formgebung kann ein federelastisches Verhalten begünstigt werden. Insbesondere können schrumpfungsbedingte Beanspruchungen des Verbindungsstegs durch ein federelastisches Verhalten in geeigneter Weise ausgeglichen werden, so dass ein ungewolltes Trennen beziehungsweise Abreißen der Membran von dem Rahmenabschnitt vermieden werden kann. Schrumpfungsbedingte Beanspruchungen können insbesondere durch Trocknungs- und/oder Sintervorgänge auftreten.

Ein voranstehend beschriebener Verbindungssteg kann auch als sogenannter Anbinder bezeichnet werden, da über diesen eine Anbindung der Membran an den Rest des Drucknutzens erfolgt.

Ein weiterer Aspekt der vorliegenden Erfindung betrifft einen Sensor, insbesondere zur transkutanen Blutgasmessung, mit einer Membran gemäß der voranstehenden Beschreibung.

Ein noch weiterer Aspekt der vorliegenden Erfindung betrifft ein Verfahren zur Herstellung einer Membran zur transkutanen Blutgasmessung, insbesondere eine voranstehend beschriebene Membran, bei dem ein Gasdurchleitungsabschnitt schichtweise durch 3D-Siebdruck erzeugt und anschließend gesintert wird, wobei zum 3D-Siebdruck eine Druckpaste mit einem metallischen oder keramischen Matrixwerkstoff sowie Formgebungselementen aus Kunststoff verwendet wird und die Formgebungselemente durch das Sintern nachträglich entfernt werden.

Ein noch weiterer Aspekt der vorliegenden Erfindung betrifft ein Verfahren zur Herstellung einer Membran zur transkutanen Blutgasmessung, insbesondere eine voranstehend beschriebene Membran, bei dem ein Gasdurchleitungsabschnitt schichtweise durch 3D-Siebdruck erzeugt und anschließend gesintert wird, wobei zum 3D-Siebdruck eine Druckpaste mit einem metallischen oder keramischen Matrixwerkstoff frei von Formgebungselementen verwendet und durch das Sintern eine poröse Struktur erzeugt wird.

Die voranstehend beschriebenen Ausführungen bezüglich der Membran gelten in gleicher Weise auch für den Drucknutzen, den Sensor sowie auch für das Verfahren zur Herstellung einer Membran.

Nachfolgend wird die Erfindung beispielhaft unter Bezugnahme auf die beigefügten Figuren beschrieben. Es zeigen, jeweils schematisch:
- Fig. 1: eine Draufsicht auf einen Drucknutzen gemäß einer Ausführungsform der vorliegenden Erfindung,
- Fig. 2: eine Membran in Detailansicht des Drucknutzend von Fig. 1 gemäß einer Ausführungsform der vorliegenden Erfindung,
- Fig. 3: eine Membran in Detailansicht eines Drucknutzens gemäß einer weiteren Ausführungsform,
- Fig. 4: eine Membran in Detailansicht eines Drucknutzens gemäß einer weiteren Ausführungsform,
- Fig. 5a: eine Schnittdarstellung eines Gasdurchleitungsabschnitts einer Membran gemäß einer Ausführungsform der vorliegenden Erfindung vor dem Sintern,
- Fig. 5b: eine Schnittdarstellung des Gasdurchleitungsabschnitts gemäß Fig. 5a nach dem Sintern,
- Fig. 6: eine Schnittdarstellung eines Gasdurchleitungsabschnitts einer Membran gemäß einer weiteren Ausführungsform der vorliegenden Erfindung nach dem Sintern.

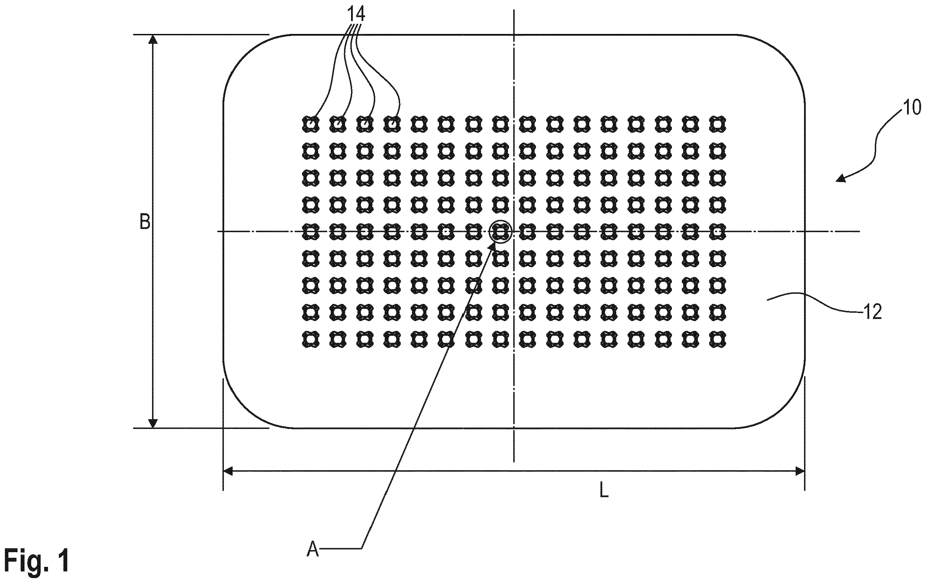

Fig. 1 zeigt eine Draufsicht auf einen Drucknutzen 10 gemäß einer Ausführungsform der vorliegenden Erfindung. Bei dem in Fig. 1 gezeigten Drucknutzen 10 handelt es sich um ein 3D-Siebdruckerzeugnis, welches auf einer spezifisch definierten Druckfläche einer 3D-Siebdruckmaschine erzeugt worden ist.

Der Drucknutzen 10 weist einen Rahmenabschnitt 12 sowie eine Mehrzahl von Membranen 14 auf. In dem Ausführungsbeispiel gemäß Fig. 1 sind insgesamt 144 Membranen innerhalb des Rahmenabschnitts 12 des Drucknutzens 10 vorgesehen. Selbstverständlich besteht auch die Möglichkeit, eine größere oder kleinere Anzahl von Membranen 14 vorzusehen. Ebenso können auch Membranen 14 mit einer anderen Dimensionierung innerhalb eines Rahmenabschnitts 12 vorgesehen sein. Die Anzahl und/oder Größe der Membrane 14 innerhalb des Rahmenabschnitts 12 können je nach Anwendungsfall geeignet ausgewählt werden.

Die Membrane 14 können in regelmäßigen Abständen zueinander innerhalb des Rahmenabschnitts 12 angeordnet sein und somit einen für das sichere Heraustrennen der Membrane 14 aus dem Rahmenabschnitt 12 geeigneten Abstand aufweisen.

Der Drucknutzen 10 kann eine Länge L von 60 bis 70 mm, insbesondere etwa 64 mm aufweisen. Ferner kann der Drucknutzen 10 eine Breite B von 40 bis 50 mm, insbesondere von 44 mm aufweisen. Der Drucknutzen 10 und somit auch jede einzelne Membran 14 kann beispielsweise eine Dicke von 0,02 mm bis 0,08 mm, insbesondere 0,03 mm bis 0,07 mm, besonders bevorzugt von 0,05 mm aufweisen.

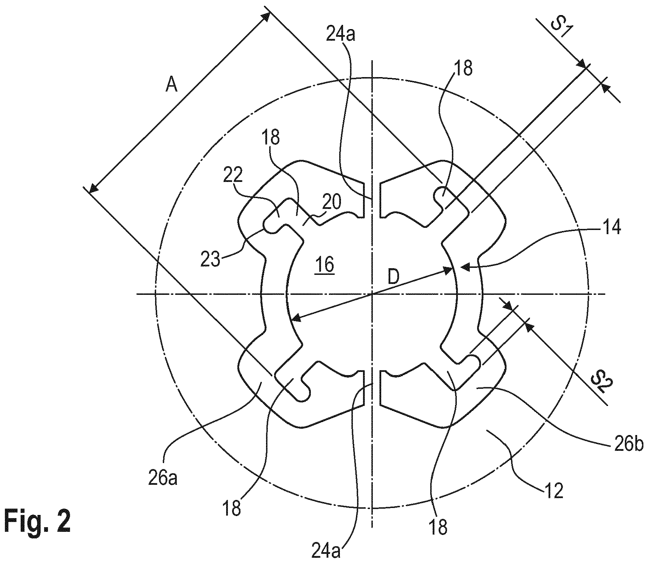

Die Fig. 2 zeigt eine Detailansicht des Drucknutzens 10. In der Detailansicht A gemäß Fig. 2 ist eine Membran 14 in einem an dem Rahmenabschnitt 12 angeordnetem Zustand gezeigt. Bei der Membran 14 handelt es sich um eine Membran 14 zur transkutanen Blutgasmessung. Eine solche Membran 14 kann insbesondere in einem hier nicht gezeigten Sensorkopf einer Sensorvorrichtung zur transkutanen Blutgasmessung angeordnet werden. Für das Messen von Sauerstoffpartialdrücken oder Kohlenstoffdioxidpartialdrücken im Blut eines Patienten kann eine Membran 14 auf der Haut angeordnet werden.

Um Partialdruckmessungen zu ermöglichen, weist die Membran 14 einen Gasdurchleitungsabschnitt 16 auf. Der Gasdurchleitungsabschnitt 16 ist durch eine poröse Struktur, insbesondere durch eine poröse Metallstruktur oder eine poröse Keramikstruktur, gebildet. Dabei kann der Gasdurchleitungsabschnitt 16 insbesondere durch eine Struktur mit offener Porosität und/oder durch eine offenzellig poröse Struktur gebildet sein. Es ist dementsprechend möglich, dass die poröse Struktur eine Vielzahl von Hohlräumen aufweist, die miteinander in Fluidverbindung stehen. Zumindest manche Hohlräume können demnach in Fluidverbindung zueinander stehen.

Für die Erzeugung von Hohlräumen innerhalb des Gasdurchleitungsabschnitts 16 beziehungsweise der Porosität der Struktur können beim 3D-Siebdruck in der jeweiligen Druckpaste Formgebungselemente - hier nicht näher dargestellt - vorgesehen sein. Zusammen mit einem Matrixwerkstoff werden diese Formgebungselemente dann durch schichtweisen Druck in die schichtweise aufzubauende Membran eingebracht.

Nach dem Drucken und Trocknen der Schichten und der Membran 14 kann dann ein Sintern erfolgen, wodurch einerseits eine Verfestigung eines metallischen Matrixwerkstoffs und gleichzeitig auch ein Ausschmelzen, Ausbrennen und/oder Ausgasen der Formgebungselemente realisiert werden kann.

Es entsteht hierdurch mit verhältnismäßig geringem Aufwand eine jeweils gewünschte Gasdurchlässigkeit beziehungsweise Permeabilität des Gasdurchleitungsabschnitts 16 beziehungsweise der gesamten Membran 14. Der Gasdurchleitungsabschnitt 16 kann durch eine solche Fertigungsweise insbesondere für Sauerstoff und/oder Kohlenstoffdioxid permeabel sein. Insbesondere kann der Gasdurchleitungsabschnitt 16 für aus der menschlichen Haut austretenden Sauerstoff und/oder Kohlenstoffdioxid permeabel sein. Dabei kann die Permeabilität beziehungsweise Gasdurchlässigkeit des Gasdurchleitungsabschnitts 16 durch die Konzentration von Formgebungselementen in der jeweiligen Druckpaste eingestellt werden.

Der Gasdurchleitungsabschnitt 16 kann einen Durchmesser D und/oder eine Diagonallänge von 0,5 mm bis 1,5 mm, insbesondere etwa 1 mm aufweisen.

Wie der Fig. 2 im Weiteren entnommen werden kann, weist die Membran 14 insgesamt vier Befestigungsabschnitte 18 auf. Die Befestigungsabschnitte 18 sind in gleichmäßigen Abständen rings um den Gasdurchleitungsabschnitt 16 herum angeordnet, also in Umfangsrichtung des Gasdurchleitungsabschnitts 16 verteilt. Jeweils zwei Befestigungsabschnitte 18 sind somit gegenüberliegend angeordnet.

Zwei gegenüberliegende Befestigungsabschnitte können in der in Fig. 2 gezeigten Draufsicht punktsymmetrisch zueinander ausgebildet sein.

Die Befestigungsabschnitte 18 können einstückig mit dem Gasdurchleitungsabschnitt 16 ausgebildet sein und/oder oder unterbrechungsfrei in den Gasdurchleitungsabschnitt 16 übergehen. Ferner können die Befestigungsabschnitte 18 relativ zu dem Gasdurchleitungsabschnitt 16 knickbar und/oder biegbar angeordnet sein. Durch Knicken und/oder Verbiegen eines Befestigungsabschnitts 18 relativ zum Gasdurchleitungsabschnitt 16 kann insbesondere eine Verformung im Übergangsbereich zwischen dem Gasdurchleitungsabschnitt 16 und dem jeweiligen Befestigungsabschnitt 18 erfolgen, was hier nicht näher dargestellt ist.

Gemäß Fig. 2 sind die Befestigungsabschnitte 18 relativ zum Gasdurchleitungsabschnitt 16 unverformt beziehungsweise unverbogen, sodass die Befestigungsabschnitte 18 relativ zum Außenumfang des Gasdurchleitungsabschnitts 16 vorstehen, insbesondere in einer Richtung der Flächenerstreckung des Gasdurchleitungsabschnitts 16 gegenüber diesem vorstehen.

Nach erfolgtem Umbiegen beziehungsweise Umknicken eines Befestigungsabschnitts 18 relativ zum Gasdurchleitungsabschnitt 16 würde der Befestigungsabschnitt 18 in einer Dickenrichtung des Gasdurchleitungsabschnitts 16 relativ zu diesem vorstehen. Ein Umknicken beziehungsweise Verbiegen eines solchen Befestigungsabschnitts 18 kann zur Befestigung der Membran 14 innerhalb einer Sensoranordnung beziehungsweise innerhalb eines Sensorkopfes erfolgen.

Der Fig. 2 kann ferner entnommen werden, dass die Befestigungsabschnitte 18 jeweils L-förmig ausgebildet sind. Hierzu weist jeder Befestigungsabschnitt 18 zwei zueinander winkelig angeordnete Schenkel 20 und 22 auf. Der Schenkel 20 erstreckt sich in radialer Richtung von dem Gasdurchleitungsabschnitt 16 weg und der Schenkel 22 kann beispielsweise unter einem rechten Winkel zum Schenkel 20 angeordnet sein. Ebenso ist es möglich, dass der Schenkel 22 in einem kleineren oder größeren Winkel von dem Schenkel 20 absteht. Der Schenkel 22 kann ein freies Ende 23 aufweisen, das gerundet ausgebildet ist.

Ferner können an dem Schenkel 20 zwei gegenüberliegend angeordnete Schenkel 22 vorgesehen sein, sodass der jeweilige Befestigungsabschnitt 18 insgesamt eine T-Form annimmt, was hier nicht näher dargestellt ist

Die Breite S1 des Schenkels 20 kann beispielsweise 0,1 mm bis 0,15 mm, insbesondere etwa 0,12 mm betragen. Die Breite S2 des Schenkels 22 kann beispielsweise 0,08 mm bis 0,15 mm, insbesondere 0,1 mm betragen.

Der Abstand A zwischen zwei freien Enden gegenüberliegender Befestigungsabschnitte 18 kann beispielsweise 1 mm bis 2 mm, insbesondere etwa 1,5 mm betragen.

Aufgrund der L-förmigen Ausgestaltung der Befestigungsabschnitte 18 kann eine in Fig. 2 gezeigte Membran auch als L-Membran bezeichnet werden. Bei einer hier nicht näher dargestellten T-förmigen Ausgestaltung der Befestigungsabschnitte 18 kann eine solche Membran auch als T-Membran bezeichnet werden.

Der Fig. 2 kann ferner entnommen werden, dass die Membran 14 über zwei gegenüberliegende Stege 24a mit dem Rahmenabschnitt 12 verbunden ist. Die Stege 24a stellen somit eine Verbindung zwischen der im 3D-Siebdruck hergestellten Membran 14 mit dem ebenfalls im 3D-Siebdruck hergestellten Rahmenabschnitt 12 sicher. Somit wird durch die Verbindung über die Stege 24a auch eine feste Positionierung der einzelnen Membrane 14 eines Drucknutzens 10 relativ zueinander gewährleistet. Derartige Verbindungsstege 24a können auch als Anbinder bezeichnet werden.

Wie in der Fig. 2 weiter zu sehen ist, ist zwischen der Membran 14 sowie dem Rahmenabschnitt 12 ein Ausschnitt 26 ausgebildet. Dieser Ausschnitt 26 ist durch die Verbindungsstege 24 unterteilt in zwei Ausschnittabschnitte 26a und 26b.

Gemäß Fig. 2 sind die Verbindungsstege 24a geradlinig verlaufend zwischen dem Gasdurchleitungsabschnitt 16 sowie dem Rahmenabschnitt 12 ausgebildet. Dabei sind die Verbindungsstege 24a mittig zwischen zwei in Umfangsrichtung benachbart angeordneten Befestigungsabschnitten 18 angeordnet.

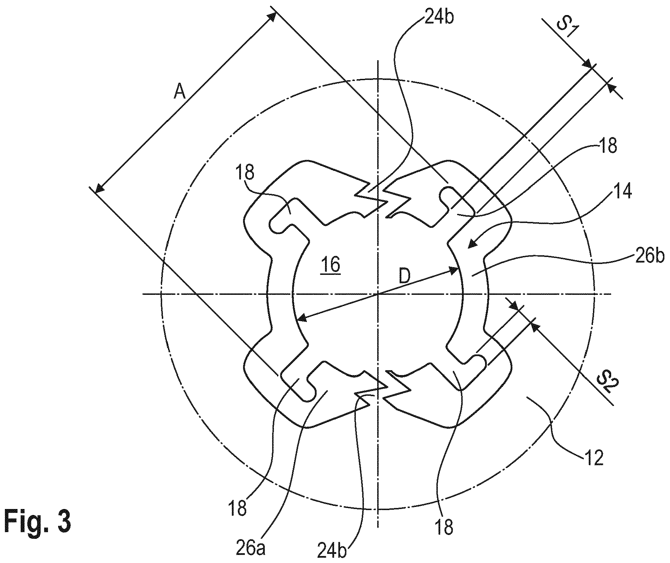

Die Fig. 3 zeigt eine alternative Ausführungsform. Die Ausführungsform gemäß Fig. 3 unterscheidet sich von der Ausführungsform gemäß Fig. 2 lediglich in Bezug auf die Verbindung zwischen der Membran 14 beziehungsweise dem Gasdurchleitungsabschnitt 16 einerseits und einem Rahmenabschnitt 12 eines Drucknutzens 10 andererseits.

Gemäß der Fig. 3 sind wiederum Verbindungsstege 24b an gegenüberliegenden Seiten des Gasdurchleitungsabschnitts 16 angeordnet. Die Verbindungsstege 24b verlaufen jedoch gezackt beziehungsweise winkelig zwischen dem Gasdurchleitungsabschnitt 16 und dem Rahmenabschnitt 12. Auf diese Weise kann durch die Verbindungsstege 24b ein verhältnismäßig hohes Maß an Federelastizität gewährleistet werden, sodass im Falle von Materialschrumpfungen, beispielsweise hervorgerufen durch den Sinterprozess, mit erhöhter Sicherheit ausgeglichen werden können. Ein unerwünschtes Abtrennen eines Materialstegs 24b durch Schrumpfungsprozesse kann auf diese Weise sicher vermieden werden.

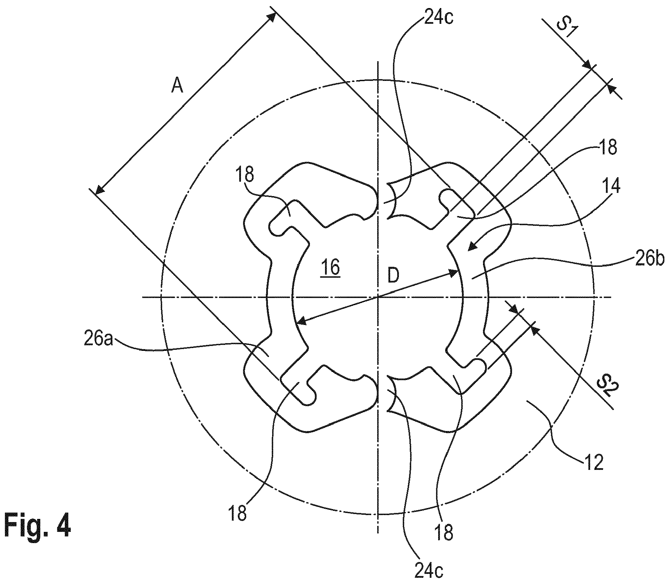

Die Fig. 4 zeigt eine weitere Ausführungsform. Die Ausführungsform gemäß Fig. 4 unterscheidet sich von den Ausführungsformen gemäß Fig. 2 oder Fig. 3 wiederum in Bezug auf die Verbindung zwischen der Membran 14 beziehungsweise dem Gasdurchleitungsabschnitt 16 einerseits und einem Rahmenabschnitt 12 eines Drucknutzens 10 andererseits.

Gemäß der Fig. 4 ist der Gasdurchleitungsabschnitt 16 wiederum durch zwei gegenüberliegend angeordnete Verbindungsstege 24c mit dem Rahmenabschnitt 12 verbunden. In der Ausführungsform gemäß Fig. 4 verlaufen die Stege 24c jedoch gekrümmt beziehungsweise gebogen zwischen dem Gasdurchleitungsabschnitt 16 und dem Rahmenabschnitt 12. Auch durch eine derart gebogene Ausgestaltung beziehungsweise durch einen gebogenen Verlauf des Verbindungsstegs 24c kann eine ausreichende Federelastizität gewährleistet werden, die etwaige Schrumpfungsprozesse, beispielsweise durch das Sintern hervorgerufen, ausgleicht. Das unerwünschte Abtrennen beziehungsweise Abreißen eines Verbindungsstegs 24c kann auf diese Weise vermieden werden.

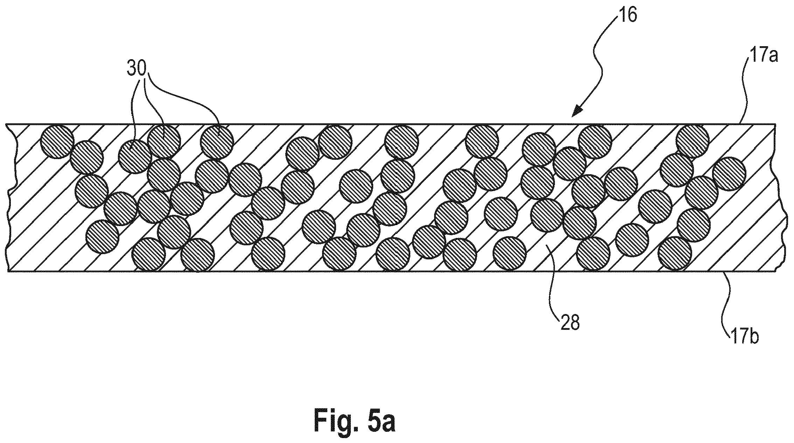

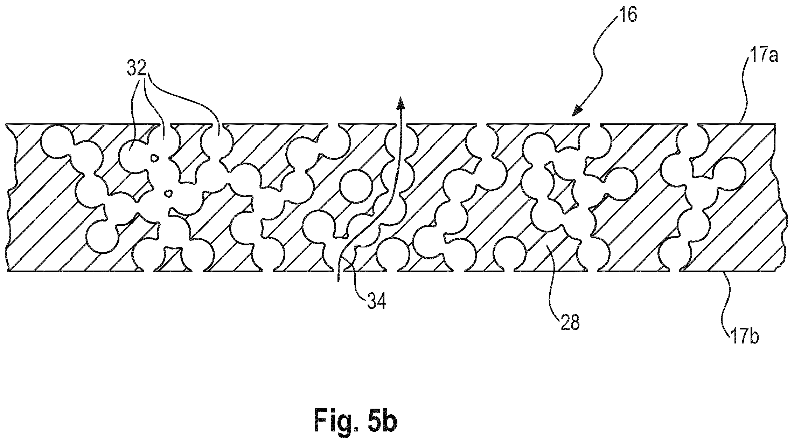

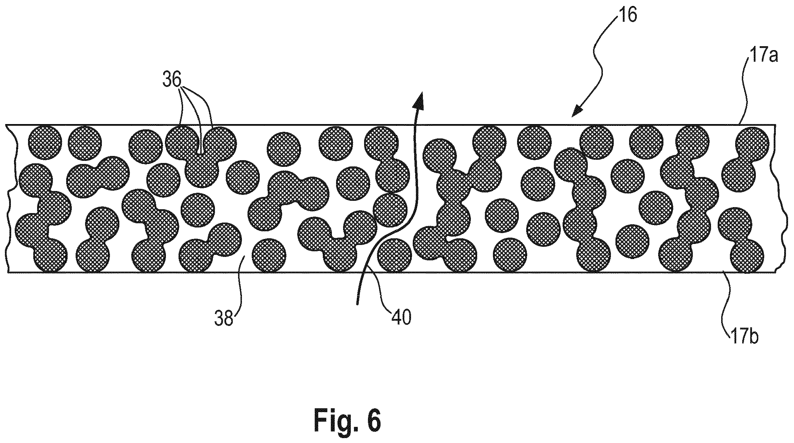

Unter Bezugnahme auf die Figuren 5a, 5b und 6 wird nachfolgend auf Einzelheiten des Gasdurchleitungsabschnitts 16 beziehungsweise der Herstellung des Gasdurchleitungsabschnitts 16 eingegangen. Dabei ist in den Fig. 5a, 5b und 6 eine Oberseite des Gasdurchleitungsabschnitts 16 mit dem Bezugszeichen 17a und eine Unterseite des Gasdurchleitungsabschnitts 16 mit dem Bezugszeichen 17b gekennzeichnet. Zwischen der Oberseite 17a und der Unterseite 17b erstreckt sich der Gasdurchleitungsabschnitt 16 in einer Dickenrichtung.

Die Fig. 5a zeigt eine Schnittdarstellung des Gasdurchleitungsabschnitts 16 einer Membran 14 gemäß einer Ausführungsform der vorliegenden Erfindung vor dem Sintern und Fig. 5b zeigt eine Schnittdarstellung des Gasdurchleitungsabschnitts 16 gemäß Fig. 5a nach dem Sintern.

In der Fig. 5a ist ein Matrixwerkstoff 28 des Gasdurchleitungsabschnitts 16 schraffiert dargestellt. Es kann sich bei dem Matrixwerkstoff 28 beispielsweise um eine Fe-Basislegierung, einen Stahlwerkstoff beziehungsweise eine Stahllegierung oder um einen Titanwerkstoff beziehungsweise eine Titanlegierung handeln. Ebenso kann es sich bei dem Matrixwerkstoff 28 um einen Keramikwerkstoff beziehungsweise um eine Keramiklegierung handeln. Innerhalb des Matrixwerkstoffs 28 ist eine Vielzahl von Formgebungselementen 30 angeordnet. Die Formgebungselemente 30 können beispielsweise kugelförmig ausgebildet sein, wie hier dargestellt. Ebenso können die Formgebungselemente 30 eckig, zylinderförmig, kegelförmig, pyramidenförmig, ellipsoid, quaderförmig, würfelförmig und/oder unregelmäßig ausgebildet sein, was hier nicht näher dargestellt ist. Die Formgebungselemente 30 können aus Kunststoff, insbesondere aus einem Polymerwerkstoff, bestehen.

Der in Fig. 5a gezeigte Zustand des Gasdurchleitungsabschnitts 16 kann beispielsweise durch schichtweisen Aufbau im Wege des 3D-Siebdruckverfahrens erfolgen. Hierzu kann eine Druckpaste eingesetzt werden, die aus dem Matrixwerkstoff 28 und den Formgebungselementen 30 besteht. Die Konzentration der Formgebungselementen 30 kann je nach Anforderungen ausgewählt beziehungsweise eingestellt sein. Nach dem schichtweisen Aufbau im Wege des 3D-Siebdruckverfahrens kann ein Sinterprozess erfolgen.

In Fig. 5b ist der Gasdurchleitungsabschnitt 16 aus Fig. 5a nach erfolgter Sinterung gezeigt. Die Formgebungselemente 30 sind durch den Sinterprozess entfernt worden, insbesondere durch Ausschmelzen, Ausbrennen oder Ausgasen, und haben eine Vielzahl von Hohlräumen 32 gebildet. Die Hohlräume 32 können dabei Negativformen oder im Wesentlichen Negativformen der entfernten Formgebungselemente 30 abbilden. Ferner können zumindest einige der Hohlräume 32 miteinander in Fluidverbindung stehen. Der Gasdurchleitungsabschnitt 16 kann somit als offenzellig poröse Struktur ausgebildet sein. Aufgrund der Fluidverbindung zwischen den Hohlräumen 32 beziehungsweise der offenzellig porösen Struktur können Strömungspfade 34 ausgebildet sein, die sich zwischen der Oberseite 17a und der Unterseite 17b erstrecken. Durch solche Strömungspfade 34 kann eine Permeabilität für Gase, insbesondere Sauerstoff und/oder Kohlenstoffdioxid, gewährleistet werden. Da gemäß dieser Ausführungsform die Hohlräume 32 durch die Entfernung der Formgebungselemente 30 erzeugt werden, können die Sinterparameter so eingestellt werden, dass zwischen den miteinander verbundenen Pulverkörnern keine oder nur kleine Hohlräume entstehen.

Fig. 6 zeigt eine Schnittdarstellung eines Gasdurchleitungsabschnitts 16 einer Membran 14 gemäß einer weiteren Ausführungsform der vorliegenden Erfindung nach dem Sintern. Bei dem Gasdurchleitungsabschnitt 16 gemäß Fig. 6 ist zur Herstellung ein Sinterwerkstoff frei von Formgebungselementen eingesetzt worden. Auch gemäß Fig. 6 kann der Gasdurchleitungsabschnitt 16 im Wege des 3d-Siebdruckverfahrens schichtweise aufgebaut werden. Hierbei kann eine Druckpaste mit einem geeigneten Sinterwerkstoff verwendet werden. Es kann sich beispielsweise um einen Sinterwerkstoff aus Metall oder Keramik handeln. Nach dem schichtweisen Aufbau des Gasdurchleitungsabschnitts 16 kann eine Sinterprozess erfolgen, bei dem eine Verfestigung erfolgt. Für das Sintern kommt vorliegend beispielsweise das Festphasensintern oder das Flüssigphasensintern in Betracht.

In der Darstellung gemäß Fig. 6 sind die einzelnen Pulverkörner 36 des Sinterwerkstoffs schematisch dargestellt. Aufgrund des Sinterprozesses sind einzelne Pulverkörner 36 des Sinterwerkstoffs miteinander verbunden. Da beim Sintern durch Einstellung geeigneter Parameter kein vollständiges Aufschmelzen des Sinterwerkstoffs erfolgt, bleiben zwischen den miteinander verbundenen Pulverkörner 36 Hohlräume 38 erhalten.

Zumindest einige der Hohlräume 38 können miteinander in Fluidverbindung stehen. Der Gasdurchleitungsabschnitt 16 gemäß Fig. 6 kann somit als offenzellig poröse Struktur ausgebildet sein. Aufgrund der Fluidverbindung zwischen den Hohlräumen 38 beziehungsweise der offenzellig porösen Struktur können Strömungspfade 40 ausgebildet sein, die sich zwischen der Oberseite 17a und der Unterseite 17b erstrecken. Durch solche Strömungspfade 40 kann eine Permeabilität für Gase, insbesondere Sauerstoff und/oder Kohlenstoffdioxid, gewährleistet werden.

In der Fig. 6 sind die Pulverkörner 36 rund beziehungsweise kugelförmig dargestellt, können aber auch jede andere beliebige Form aufweisen.

Eine vorstehend beschriebene Membran 14 eignet sich insbesondere für die transkutane Blutgasmessung beziehungsweise für den Einsatz in einem Sensorkopf zur transkutanen Blutgasmessung. Über die L-förmigen Befestigungsabschnitte 18 kann eine geeignete Befestigung innerhalb eines Sensorkopfes vorgenommen werden. Hierzu können die Verbindungsabschnitte 18 zerstörungsfrei verbogen beziehungsweise geknickt werden. Ein einfacher Austausch wird durch die zerstörungsfreie Biegbarkeit beziehungsweise Knickbarkeit der Verbindungsabschnitte 18 ermöglicht.

Durch den schichtweisen Aufbau im 3D-Siebdruckverfahren, dem gleichzeitigen Einbringen von Formgebungselementen sowie der nachträglichen Entfernung von Formgebungselementen aus dem jeweiligen metallischen Matrixwerkstoff durch einen Sinterprozess kann eine effiziente Herstellung gewährleistet werden. Insbesondere können aufwendige Nachbearbeitungen durch Laserbohrungen oder dergleichen vermieden werden. Gleichzeitig wird durch eine derart hergestellte Membran 14 ein hohes Maß an Gasdurchlässigkeit beziehungsweise Permeabilität sichergestellt.

Eine erfindungsgemäße Membran 14 beziehungsweise ein Sensor mit einer solchen Membran 14 eignet sich insbesondere zur transkutanen Blutgasmessung in der Überwachung von Neugeborenen.

## Patentansprüche

1. Membran (14) zur transkutanen Blutgasmessung, mit wenigstens einem Gasdurchleitungsabschnitt (16), der durch eine poröse Struktur gebildet ist.

2. Membran (14) nach Anspruch 1,
**dadurch gekennzeichnet, dass**
der Gasdurchleitungsabschnitt (16) durch eine poröse Metallstruktur oder durch eine poröse Keramikstruktur gebildet ist.

3. Membran (14) nach Anspruch 1 oder 2,
**dadurch gekennzeichnet, dass**
der Gasdurchleitungsabschnitt (16) durch eine Struktur mit offener Porosität und/oder durch eine offenzellig poröse Struktur gebildet ist und/oder dass die poröse Struktur eine Vielzahl von miteinander in Fluidverbindung stehenden Hohlräumen aufweist.

4. Membran (14) nach zumindest einem der vorstehenden Ansprüche,
**dadurch gekennzeichnet, dass**
Hohlräume (32, 38) der porösen Struktur Negativformen von nachträglich entfernten Formgebungselementen (30) abbilden.

5. Membran (14) nach zumindest einem der vorstehenden Ansprüche,
**dadurch gekennzeichnet, dass**
die poröse Struktur hergestellt ist durch Erzeugung eines Materialabschnitts aus einem metallischen und/oder keramischen Matrixwerkstoff (28) mit darin angeordneten Formgebungselementen (30) aus Kunststoff und durch Erhitzen des Materialabschnitts zur nachträglichen Entfernung der Formgebungselemente (30).

6. Membran (14) nach zumindest einem der vorstehenden Ansprüche,
**dadurch gekennzeichnet, dass**
die poröse Struktur gesintert ist und/oder die Porosität durch Sintern erzeugt ist und/oder dass die poröse Struktur durch Sintern eines metallischen oder keramischen Matrixwerkstoffs (28) mit darin angeordneten Formgebungselementen (30) erzeugt ist und/oder dass die Formgebungselemente (30) durch Ausschmelzen, Ausbrennen und/oder Ausgasen entfernt sind und/oder dass die Formgebungselemente (30) durch gleichzeitiges Sintern eines metallischen oder keramischen Matrixwerkstoffs (28) entfernt sind.

7. Membran (14) nach zumindest einem der vorstehenden Ansprüche,
**dadurch gekennzeichnet, dass**
die poröse Struktur durch Sintern eines metallischen oder keramischen Werkstoffs frei von gesonderten Formgebungselementen erzeugt ist.

8. Membran (14) nach zumindest einem der vorstehenden Ansprüche,
**dadurch gekennzeichnet, dass**
der Gasdurchleitungsabschnitt (16) durch additive Fertigung erzeugt ist, insbesondere durch 3D-Siebruck, und/oder dass der Gasdurchleitungsabschnitt (16) aus einer Druckpaste mit Formgebungselementen aus Kunststoff gedruckt und nachträglich gesintert ist und/oder dass der Gasdurchleitungsabschnitt (16) durch eine Mehrzahl an Schichten erzeugt ist.

9. Membran (14) nach zumindest einem der vorstehenden Ansprüche,
**dadurch gekennzeichnet, dass**
dass der Gasdurchleitungsabschnitt (16) permeabel ist, zumindest für Sauerstoff und/oder Kohlenstoffdioxid permeabel ist, und/oder dass der Gasdurchleitungsabschnitt (16) für aus der menschlichen Haut austretenden Sauerstoff und/oder Kohlenstoffdioxid permeabel ist.

10. Membran (14) nach zumindest einem der vorstehenden Ansprüche,
**dadurch gekennzeichnet, dass**
der Gasdurchleitungsabschnitt (16) eine eingestellte Porosität aufweist, insbesondere eine Porosität von 0,1 bis 0,9, bevorzugt von 0,2 bis 0,8, weiter bevorzugt von 0,3 bis 0,7, noch weiter bevorzugt von 0,4 bis 0,6, und/oder dass die Porosität des Gasdurchleitungsabschnitts (16) durch die Konzentration von Formgebungselementen (30) eingestellt ist und/oder dass der Gasdurchleitungsabschnitt (16) eine eingestellte Permeabilität aufweist und/oder dass die Permeabilität des Gasdurchleitungsabschnitts (16) durch die Konzentration von Formgebungselementen (30) eingestellt ist.

11. Membran (14) nach zumindest einem der vorstehenden Ansprüche,
**dadurch gekennzeichnet, dass**
der Gasdurchleitungsabschnitt (16) eine Dicke von 0,01 mm bis 1 mm, bevorzugt von 0,02 mm bis 0,08 mm, weiter bevorzugt von 0,03 mm bis 0,07 mm, noch weiter bevorzugt von 0,04 mm bis 0,06 mm, insbesondere etwa 0,05 mm aufweist und/oder dass der Gasdurchleitungsabschnitt (16) einen Durchmesser und/oder eine Diagonallänge von 0,5 mm bis 1,5 mm, bevorzugt von 0,7 mm bis 1,3 mm, weiter bevorzugt von 0,9 mm bis 1,1 mm, insbesondere etwa 1 mm aufweist.

12. Membran (14) nach zumindest einem der vorstehenden Ansprüche,
**dadurch gekennzeichnet, dass**
zumindest ein Befestigungsabschnitt (18) zur Befestigung des Gasdurchleitungsabschnitts (16) an einem Sensor zur transkutanen Blutgasmessung vorgesehen ist und/oder dass eine Mehrzahl von Befestigungsabschnitten (18) vorgesehen ist und/oder dass der Befestigungsabschnitt (18) einstückig mit dem Gasdurchleitungsabschnitt (16) ausgebildet ist und/oder an den Gasdurchleitungsabschnitt (16) angrenzt und/oder dass eine Mehrzahl an Befestigungsabschnitten (18) um den Gasdurchleitungsabschnitt (16) herum angeordnet ist, insbesondere gleichmäßig um den Gasdurchleitungsabschnitt (16) herum angeordnet ist.

13. Membran (14) nach Anspruch 12,
**dadurch gekennzeichnet, dass**
der Befestigungsabschnitt (18) relativ zum Gasdurchleitungsabschnitt (16) knickbar und/oder biegbar ist, insbesondere zerstörungsfrei relativ zum Gasdurchleitungsabschnitt (16) knickbar und/oder biegbar ist, und/oder dass der Befestigungsabschnitt (18) gegenüber dem Gasdurchleitungsabschnitt (16) vorsteht, insbesondere in einer ungeknickten und/oder unverbogenen Ausgangsposition gegenüber einer Außenkontur des Gasdurchleitungsabschnitts (16) vorsteht und/oder in einer geknickten oder gebogenen Stellung in Dickenrichtung gegenüber dem Gasdurchleitungsabschnitt (16) vorsteht.

14. Membran (14) nach Anspruch 12 oder 13,
**dadurch gekennzeichnet, dass**
dass der Befestigungsabschnitt (18) L-förmig oder T-förmig ausgebildet ist und/oder zur formschlüssigen Befestigung und/oder zum Hintergreifen einer an einem Sensor vorgesehen Eingriffsstruktur ausgebildet ist und/oder dass der größtmögliche Abstand zwischen zwei freien Enden gegenüberliegender Befestigungsabschnitte (18) zwischen 1 mm und 2 mm beträgt, insbesondere zwischen 1,2 mm und 1,8 mm, bevorzugt etwa 1,5 mm und/oder dass der Gasdurchleitungsabschnitt (16) unterbrechungsfrei in den Befestigungsabschnitt (18) übergeht und/oder dass der Gasdurchleitungsabschnitt (16) und der Befestigungsabschnitt (18) eine identische Lagenstruktur und/oder eine identische Dicke aufweisen und/oder gemeinsam durch 3D-Siebdruck erzeugt sind.

15. Drucknutzen (10), insbesondere 3D-Siebdrucknutzen, mit einem Rahmenabschnitt (12) und einer Mehrzahl von Membranen (14) nach einem der vorstehenden Ansprüche, wobei jeweils eine Membran (14) durch zumindest einen Verbindungssteg (24a, 24b, 24c) mit dem Rahmenabschnitt (12) verbunden ist.

16. Sensor, insbesondere zur transkutanen Blutgasmessung, mit einer Membran (14) nach einem der vorstehenden Ansprüche 1 bis 14.

17. Verfahren zur Herstellung einer Membran (14) zur transkutanen Blutgasmessung, insbesondere eine Membran (14) nach einem der Ansprüche 1 bis 14, bei dem ein Gasdurchleitungsabschnitt (16) schichtweise durch 3D-Siebdruck erzeugt und anschließend gesintert wird, wobei zum 3D-Siebdruck eine Druckpaste mit einem metallischen oder keramischen Matrixwerkstoff (28) sowie Formgebungselementen (30) aus einem Kunststoff verwendet wird und die Formgebungselemente (30) durch das Sintern nachträglich entfernt werden.
